# EUROPEAN PATENT SPECIFICATION

(11) **EP 0 497 956 B1**
(45) Date of publication and mention of the grant of the patent: **01.05.1996**
(21) Application number: 91915434.4
(22) Date of filing: 20.08.1991
(51) Int. Cl.: A61K 9/14

(54) **CONTROLLED RELEASE FORMULATIONS**
ZUSAMMENSETZUNGEN ZUR KONTROLLIERTEN FREIGABE
FORMULATIONS DE LIBERATION CONTROLEE

(30) Priority: 21.08.1990 US 570340; 11.09.1990 US 580854; 01.04.1991 US 678814
(43) Date of publication of application: 12.08.1992
(73) Proprietor: TheraTech, Inc., Salt Lake City, Utah 84108 (US)
(72) Inventor: ACHARYA, Ramesh, N., Lake Forest, IL 60045 (US)
(74) Representative: Brewster, Andrea Ruth
(86) International application number: US9105955
(87) International publication number: WO9203124

(56) References cited:
- EP-A- 0 371 421
- WO-A-89/06964
- WO-A-91/06283
- US-A- 4 615 697
- US-A- 4 988 679

## Description

### FIELD OF THE INVENTION

This invention relates to certain controlled or sustained dosage forms and, in particular, to certain polymeric matrices or complexes which are suitable for achieving controlled or sustained delivery of an active composition, such as cosmetic agents, nutritional agents, household agents, agricultural agents and industrial chemical agents.

### BACKGROUND OF THE INVENTION

Delivery of pharmaceutical compositions through the use of polymeric carriers is a well known technique. U.S. Patent No. 4,615,697 to Robinson provides an excellent review of this subject, especially as it relates to the use of bioadhesive compositions for buccal administration. The buccal delivery systems disclosed in Robinson utilize known bioadhesives to hold the polymeric system in place in the buccal cavity after insertion therein. The drug is released from a bioadhesive matrix and absorbed into the buccal lining. The compositions disclosed in Robinson provide a means of trans-mucosal delivery of therapeutic agents that are subject to poor bioavailability due to solubility limitations, polarity considerations, degradation due to pH, enzymatic exposure or "first pass" metabolism by the liver or gastrointestinal enzymes after oral ingestion. However, such delivery systems, although of general utility have certain disadvantages in actual application. U.S. Patent No. 4,900,552 provides a composition for releasing active ingredients in the buccal cavity itself for an extended period of time. The composition of that patent comprises a trilaminate film segment capable of delivering an active ingredient within the buccal cavity while attached to a wall of that cavity. The trilaminate film segment includes a hydratable bioadhesive base layer, a non-adhesive reservoir layer and a water-impermeable barrier sandwiched between and bonded to the base layer and the reservoir layer. Such a composition is by its very nature a complex structure requiring detailed formulation techniques to achieve the desired composition.

Alginic acid, including its salts, has also been used in various forms and combinations for purposes of providing bioadhesive compositions for the administration of active compositions. As one example thereof, the use of cross-linked alginate gum gel is described in U.S. Patent No. 3,640,741 to Etes as being suitable for use as the bioadhesive.

As an alternative to the bioadhesive approach, lozenges, candies, lolipops, chewing gums and the like are often used to deliver active compositions. Typically there is entrained an active agent in a slowly dissolving or disintegrating material, such as common and complex carbohydrates, starches, natural and synthetic polymers and the like. With respect to the types of delivery systems which rely on solvation or disintegration, the active composition is released as the matrix dissolves or disintegrates after contact with the saliva.

For chewing gum delivery systems, a rubber-like polymer, such as a polybutadiene typically is used, which does not dissolve nor disintegrate in the mouth. Release of the active composition is through diffusion and migration of the active composition through the polymeric matrix to the surface of the product as a result of the chewing and mastication action, causing ultimately mixing of the active composition with the saliva.

Both of the aforementioned types of delivery systems have the disadvantage that the rate of release of the active composition is highly dependent upon the chewing action of the individual. Further, the presence of sugars and the chewing action stimulates saliva secretion which in turn results in limited effective release times. Further, the presence of high levels of sugars in the formulations promote bacterial growth and cause dental and peridontal diseases.

Document EP-A-0 371 421 discloses calcium polycarbophil that adheres to mucous membranes.

Because of all of the foregoing shortcomings in the prior art delivery methods, a need has existed for a delivery system suitable for oral, buccal and gingival delivery of active compositions that overcomes such deficiencies in the prior art systems.

There is also a considerable interest in developing controlled release delivery systems suitable for parenteral applications. A variety of sophisticated approaches such as biodegradable implants, liposomes, injectable microspheres, injectable microsponges, and "self depot" injections have been reported in the literature. In all of these types of controlled release delivery systems, there are numerous limitations. A need has existed for delivery systems which can be manufactured easily and administered parenterally using currently available administration systems. With the present invention it is possible to design a delivery system which is fluid at the time of injection but polymerizes in the body to form a hydrogel matrix, to achieve controlled release of active ingredients over a period from a few days to many months.

Burn treatments and wound healing applications require special delivery systems for local application of active agents. In these conditions the affected local area is highly compromised and minimum additional trauma can be tolerated. Liquid preparations, sprays, gels, medicated bandages and liquid skins have been reported in the literature to provide controlled local delivery of active agents, each in their own deficiencies. Additional requirements are introduced by many dermatological and ophthalmic conditions e.g. psoriasis, dermatitis and ocular infections and the like, which require controlled delivery systems for a specific site or for a large area of the body, for local pharmacological actions.

Another application area of concern is the site specific and target-organ delivery of chemotherapeutics and radiological agents. Effective treatment in these applications require achievement of a very high concentration of active agent in specific sites, e.g. tumor cells, hyperactive glands and the like. "Depot" delivery or "delivery depot" processed by external devices, such as magnetic focusing are reported to contain the drug within the target organ but the complexities of the known techniques introduce additional undesirable complications and requirements.

There also exists a need for controlled or sustained release of cosmetic agents, such as sunscreens, and the like, to provide such cosmetic agents with improved properties and utilities.

Similarly, there exists a need for controlled or sustained release of agricultural agents, such as herbicides and fertilizers, and household agents, such as bleaches and room fresheners, all with the goal of providing such agents with improved properties and utilities.

An additional need to which the present invention is directed is for a composition for treating the physiological condition known as "dry mouth," a condition that causes discomfort and difficulty in sleeping, also known as xerostomia. Currently, cotton balls, sponges, and the like are used in attempts to alleviate the discomfort of dry mouth, but such approaches are not desirable as it is possible that the cotton or sponge may be inadvertently swallowed, as during sleeping.

### SUMMARY OF THE INVENTION

The present invention provides, in one aspect, a polymeric delivery system which is formed through the use of a calcium polycarbophil type composition with an active agent, optionally in the presence of water and/or other cosolvent. The resulting product is especially useful as a carrier or coating of active compositions, such as pharmaceuticals and the like, and as a coating for pharmaceutical excipients, actives and intermediate products, to retard dissolution of a substrate e.g. granules, sugar crystals, tablets and the like, and provides a means for achieving a rate-controlled release of the active composition. The present invention provides several advantages and benefits, including an improved composition and method for the controlled release of an active composition, such as a pharmaceutical, to oral, buccal or gingival skin or mucosa over a period of time. The compositions for application to the skin are not noticeably irritating to the skin or mucosa with which they are contacted and they may contain substantially any medicinal agent or cosmetic agent.

The present invention is also related to a method of controlled release treatment by use of a polymeric carrier containing a therapeutically effective amount of an active composition, wherein the polymeric complex carrier is formed by the interaction of a calcium polycarbophil type component with the active composition, optionally in the presence of water, and is then used, for example, to contact an area of skin or mucous membrane to be treated with said active composition, for a sufficient period of time to allow a therapeutically effective amount of said active composition to be released from the matrix.

The present invention is further related to a method of controlled release treatment through use of a polymeric carrier and a therapeutically effective amount of an active composition which is a "complex hydrogel" that is formed within the body after it is administered, or at the site of application, to provide controlled release of an active agent from the matrix over the course of a few days to a few months. The composition may be supplied as a two part system, a polymer phase and a liquid phase. Upon reconstitution of the two phases, the system initially remains in a fluid state to allow its delivery internally. Once injected, it forms a highly structured hydrogel matrix within short time to provide a controlled delivery of active agents, such as drugs dispersed within the matrix.

The present invention additionally provides a delivery system for controlled release at local applications. The treatment comprises a pourable liquid preparation, or a powder which upon contact with water, that sets within a few seconds after it is applied locally. In one aspect, the delivery system may also consist of a two part system which comprises a polymer phase and a liquid phase. The active medicinal agents may be present in either or both the phases. The two parts may be thoroughly mixed prior to application. Some preparations of the present invention may have a thick consistency, enabling them to be poured over the area to be treated, forming a controlled release hydrogel system. Such a system allows the drug dispersed within the matrix to diffuse to the local site of application. Because of the hydrogel nature of the matrix, the protective barrier allows free diffusion of materials from the local sites into the matrix and thus behaves as a breathable protective barrier.

The present invention, in another aspect, is related to a method of targeting administration of chemotherapeutic agents and radiologicals at the desired sites, e.g. cancerous tumors, hyperactive glands, and the like, using compositions containing an active agent and excipients to form special "implant" type devices. The implant device will polymerize upon contact with water to form a complex matrix system to control the release of the active agent. The system may optionally consist of a two-part system, a polymer and liquid phase. When the two parts are mixed together, the system remains fluid for a short period of time to allow its administration. Once at the site of application, it completes its internal structuration to form a complex hydrogel matrix containing the active drug within the area of the target site. The polymeric delivery system comprising calcium polycarbophil and other excipients, along with active agents, may be formulated with non-polar solvents and cosolvents to maintain its fluidity. Once injected, intercellular and/or intracellular water will penetrate and activate the hydrogel matrix formations to achieve controlled drug delivery systems.

With the present invention it is possible to design a delivery system which is fluid at the time of injection but polymerises in the body to form a hydrogel matrix, to achieve controlled release of active ingredients over a period from a few days to many months.

Also as indicated above, the present invention functions to provide compositions for the controlled or sustained release of active agents that are cosmetic agents, household agents, agricultural agents, industrial chemical agents and nutritional agents. Such compositions may be used as in their normal manner of application, but provide advantageous results in the form of controlled or sustained release or the active agent. Thus, the compositions of the present invention containing such active ingredients may have longer useful lives or may function to reduce the toxicity of the active agent that is incorporated therein.

Additionally, the present invention provides a composition for treating "dry mouth" or xerostomia. The composition comprises polycarbophil, in any form suitable for insertion into the mouth, such as a wafer. The polycarbophil is preferably hydrated prior to insertion into the mouth.

In another aspect of the present invention, bioadhesive compositions are provided that are polymeric complexes that are formed through the interaction of a polycarbophil type composition, or a salt thereof, with alginic acid, or a salt thereof, in the presence of a divalent cation. Because of the nature of such polymeric complexes of the present invention, they exhibit some bioadhesive properties. Such polymeric complexes also may be made to contain an active composition, such as a pharmaceutical, by incorporating such an agent into either the polycarbophil component or the alginic acid component, prior to forming the complex between the two constituents.

The present invention thus is also related to a method of controlled release treatment comprising providing a polymeric complex carrier and a therapeutically effective amount of an active composition, wherein the polymeric complex carrier is formed by the interaction of a polycarbophil component with an alginic acid component, in the presence of a divalent cation and the active composition, and contacting an area of skin or mucous membrane to be treated with said composition for a sufficient period of time to allow a therapeutically effective amount of said active composition to be released from the complex.

Other benefits and advantages of the present invention will be apparent to those skilled in the art from the Detailed Description, Examples and Claims that follow.

The invention is defined by the appended claims.

### DETAILED DESCRIPTION OF THE PREFERRED EMBODIMENTS

### COMPOSITIONS FORMED FROM CALCIUM POLYCARBOPHIL - ACTIVE AGENT - AND (OPTIONALLY) WATER

The present invention relates to a polymeric delivery system which may be used as controlled release carriers, and to methods for the use of such systems. The delivery systems of the present invention are formed by the use of a calcium polycarbophil type composition with an active agent, optionally in the presence of water. Preferably, the calcium cation is initially present in the form of a salt with the polycarbophil type composition.

When water is present, the matrix of the present invention provides the controlled release effect by forming a complex hydrogel which is gum/sponge-like in consistency and retards the dissolution and diffusion of the active ingredient from the matrix which, for example, may be in the form of a tablet, lozenge, candy, granule, suppository or the like. The hydrogel matrix system may be formed at the time of application and such an approach is especially useful for treating dermatological and ocular disorders, as through burn and wound healing treatments, ocular infections, and ocular inflammations, and for providing parenteral or local application of active agents.

Calcium polycarbophil, upon contact with water and optionally with a cosolvent, sets in a matter of a few minutes into a cohesive hydrogel material which may be either a rubbery mass, or a highly rigid mass, for example, if the requisite amount of water is added. Such a cohesive material may be of rubber/sponge-like consistency and does not have any inherent taste nor odor. Further, the composition does not readily dissolve nor disintegrate when exposed to saliva and can withstand a mild amount of chewing or mastication. Further, the composition can control the release of soluble materials by retarding their dissolution and diffusion within and from the matrix.

The resulting matrix of calcium polycarbophil, active agent and water may be a polymeric hydrogel matrix which may be formed, as by cutting, into small pieces of appropriate size and shape. The resulting product can be dried to any degree of hardness and moisture content. As an alternative, the calcium polycarbophil may simply be mixed with active agents and with other excipients, if desired, to form desirable compositions. The calcium polycarbophil may also be mixed with water, with or without other cosolvents, or may be mixed with organic solvents, without water, then granulated, dried, if necessary, to a desired initial dry weight moisture content and tableted using conventional tableting procedures. The resultant products may be molded into lozenges, suppositories or gums, for example; encapsulated into gelatin capsules; compressed into tablets; ground into dry powders or granules and may be further coated, if desired.

Additionally, the compositions of the present invention, when formed at the time of administration, may be used as in the form of an ingested or implanted bolus, as through parenteral injection, which upon administration will controllably release the active composition with passage of time only.

The interaction of the calcium polycarbophil, optionally in the presence of water, a hydrocolloid or polyols or cosolvents, with an active agent, results in the formation of a polymeric hydrogel complex of varying solubility which affects the dissolution of the active agent out of the matrix. The controlled release rate of the active agent is dependent, in part, upon the quantity of water and/or other cosolvents initially present to form the complex, if any, and in part upon the interaction of the polymer and other excipients, including the hydrocolloids, and/or carbohydrates if present. Active agents, such as medicaments, may be released by diffusion or leaching through the sponge matrix or by erosion of the matrix. An active agent, such as a medicament, may be released in a controlled manner for extended periods up to several months. Typically, the duration may be for several weeks, in the case of implanted boluses and the like, to several days, such as from 1 to 3 days. In orally administered chewable products, the duration of release usually is for at least about four to twelve hours, and typically for about one-half hour or more.

### BIOADHESIVE COMPOSITIONS CONTAINING ALGINIC ACID COMPONENTS

When bioadhesive compositions are desired, polymeric complexes may be formed through interaction of a polycarbophil type composition with an alginic acid component, in the presence of a divalent cation. Preferably, the divalent cation is initially present in the form of a salt with the polycarbophil type composition. The compositions also typically include an effective amount of an active composition which is incorporated in the polymeric complex. As indicated, due to the structure of the polymeric complexes of the present invention, the complexes may also be considered to be bioadhesives. Varying degrees of bioadhesive properties are exhibited by the complexes, as the alginic acid component is itself a bioadhesive. Calcium polycarbophil, which is the preferred form of the polycarbophil component, has no bioadhesive properties.

The term bioadhesive, as used herein, is a material that adheres to a mucosal tissue surface in-vivo and/or in-vitro upon hydration. Such adhesion will adherently localize the polymeric complex onto the mucus membrane.

The compositions of the present invention made with the use of alginic acid are designed for use on the skin and mucus membranes (mucosa) of an animal body, such as that of a human, to which the compositions adhere in the presence of a sufficient amount of water to swell the compositions. The compositions, when containing an active composition, are adhered to mucosa or skin and controllably release the active composition to the contacted body area for a period of time, and cause the active composition to be sorbed (absorbed or adsorbed) at least at the vicinity of the contacted body area.

### ACTIVE COMPOSITIONS

The active compositions useful herein are selected generally from the classes of medicinal agents, cosmetic agents, nutritional agents, household agents, industrial chemical agents, and agricultural agents. Substantially any such agent may be used in the present invention including both solid and liquid active compositions.

The active composition may be used singly or as a mixture of two or more such agents.

When the active agent employed in the present invention is a household agent, it may be, for example, an air freshener, a bleach, such as a chlorine bleach, a cleaner, a fishtank preservative or the like.

When the active agent employed in the present invention is a cosmetic agent, it may be, for example, a sunscreen agent, a skin softener, a moisturizing agent, an emollient, or the like.

When the active agent employed in the present invention is a nutritional agent it may be a vitamin or mineral or a complex thereof.

When the active agent is an agricultural agent, it may be, for example, an herbicide, a pesticide, a fungicide, a rodenticide, a plant nutrient, or a growth hormone or a combination of one or more such agents.

Thus, the active composition may be any medicinal agent, such as an agent for treating an internal condition, an agent for treating a mental health condition, an antibiotic active composition, a chemotherapeutic agent, an anti-inflammatory agent, a high molecular weight protein or polypeptide treating agent, or the like.

Exemplary medicinal agents include agents for treating cardiovascular conditions such as chlorothiazide (diuretic), propranolol (antihypertensive), hydralazine (peripheral vasodilator), isosorbide or nitroglycerin (coronary vasodilators), metoprolol (beta blocker), procainamide (antiarrythmic), clofibrate (cholesterol reducer) or coumadin (anticoagulant); agents for treating internal conditions such as conjugated estrogen (hormone), tolbutamide (antidiabetic), levothyroxine (thyroid conditions), propantheline (antispasmodic), cimetidine (antacid), phenyl propanolamine (antiobesity), atropine or diphenoxalate (antidiarrheal agents), docusate (laxative), or prochlorperazine (antinauseant); agents for treating mental health conditions such as haloperidol or chlorpromazine (tranquilizers), doxepin (psychostimulant), phenytoin anticonvulsant), levo dopa (antiparkinism), benzodiazepine (antianxiety) or phenobarbital (sedative); anti-inflammatory agents such as fluorometholone, acetaminophen, phenacetin, aspirin, hydrocortisone, or predisone; anti-histamines such as diphenhydramine hydrochloride or dexchlorpheniramine maleate; antibiotics such as sulfanilamide, sulfamethizole, tetracycline hydrochloride, penicillin and its derivatives, cephalosporin derivatives or erythromycin; chemotherapeutic agents such as sulfathiazole, doxorubicin, cisplatin or nitrofurazone; topical anesthetics such as benzocaine; cardiac tonics such as digitalis or digoxin; antitussives and expectorants such as codeine phosphate, dextromethorphan or isoproterenol hydrochloride; oral antiseptics such as chlor hexidine hydrochloride or hexylresorcinol; enzymes such as lysozyme hydrochloride or dextronase; birth control agents such as estrogen; opthalmic treating agents such as timolol or gentamycin, and the like. In addition, medicinal treating agents may also include whole proteins such as the VP3 capsid protein (also known as the VP Thr and VP1 capsid proteins in other nomenclature systems) of foot-and-mouth disease virus described in U.S. Pat. No. 4,140,763 as being useful as the active ingredient in a vaccine against foot-and-mouth disease, insulin or interferon; polypeptide treating agents such as endorphins, human growth hormone, or bovine growth hormone, or still lower molecular weight polypeptides or conjugates of those polypeptides linked protein carriers as are described in Sutcliffe et al., Science, 219, 660-666 (1983).

Medicaments which are orally administered in accordance with the present invention include, for example, adrenergic agents such as ephedrine, desoxyephedrine, phenylephrine, epinephrine and the like, cholinergic agents such as physostigmine, neostigmine and the like, antispasmodic agents such as atropine, methantheline, papaverine and the like, curariform agents such as chlorisondamine and the like, tranquilizers and muscle relaxants such as fluphenazine, chlorpromazine, triflupromazine, mephenesin, meprobamate and the like, antidepressants like amitriptyline, nortriptyline, and the like, antihistamines such as diphenhydramine, dimenhydrinate, tripelennamine, perphenazine, chlorprophenazine, chlorprophenpyridamine and the like, hypotensive agents such as rauwolfia, reserpine and the like, cardioactive agents such as bendroflumethiazide, flumethiazide, chlorothiazide, aminotrate, propranolol, nadolol, metoprolol, atenolol, procainamide and the like, angiotensin converting enzyme inhibitors such as captopril and enalapril, bronchodialators such as theophylline, steroids such as testosterone, prednisolone, and the like, antibacterial agents, e.g., sulfonamides such as sulfadiazine, sulfamerazine, sulfamethazine, sulfisoxazole and the like, antimalarials such as chloroquine and the like, antibiotics such as the tetracyclines, nystatin, erythromycin, streptomycin, cephradine and other cephalosporins, penicillin, semisynthetic penicillins, griseofulvin and the like, sedatives such as chloral hydrate, phenobarbital and other barbiturates, glutethimide, antitubercular agents such as isoniazid and the like, analgesics such as aspirin, acetominophen, propoxyphene, meperidine, ibuprofen, and the like, etc. These substances are frequently employed either as the free compound or in a salt form, e.g., acid addition salts, basic salts like alkali metal salts, etc. Other therapeutic agents having the same or different physiological activity can also be employed in pharmaceutical preparations within the scope of the present invention.

### OPTIONAL COMPONENTS

The controlled release rate of the active agent is dependent upon the structure of the polymeric matrix which may be modified through use of water, polar and nonpolar cosolvents, and by varying their amounts and the inclusion of other components, e.g. carbohydrates and hydrocolloids which act to modify the physical and chemical properties of the matrix. For example, an auxiliary hydrocolloid may be employed, such as cellulose polymers which are cellulose ethers such as methyl cellulose, cellulose alkyl hydroxylates such as hydroxypropylmethyl cellulose, hydroxypropyl cellulose, hydroxymethyl cellulose or hydroxyethyl cellulose, cellulose alkyl carboxylates such as carboxymethyl cellulose and carboxyethyl cellulose, and alkali metal salts of cellulose alkyl carboxylates, such as sodium carboxymethyl cellulose and sodium carboxyethyl cellulose, as well as carboxypolymethylene (molecular weight 2.5 to 3.5 million), gum acacia, guar gum, gum tragacanth, gum xanthan, alkali metal or alkaline earth metal carageenates, and alginates, such as alginic acid, ammonium or sodium alginate or mixtures thereof. Simple or complex carbohydrates or polyols, such as sucrose, xylose, mannitol, glucose, starch, Pluronic® surfactants, inorganic salts such as dicalcium phosphate, and the like, may also be employed to modify the hydrogel stucture.

Other ingredients that can be present in the compositions of the present invention include breath fresheners and flavors, e.g., spearmint oil, peppermint oil, cinnamaldehyde, cetyl pyridinium chloride, menthol saccharin sodium, glycyrrhizin, malt syrup, citric acid, tartaric acid, lemon oil, citrus flavor, and the like, sodium fluoride and the like, anti-plaque/anti-bacterial compositions suitable to treat or prevent periodontal disease, e.g., chlorobutanol, chlorothymol, chlorohexidine, their salts, and the like, dental pain control ingredients, e.g., benzocaine, lidocaine and the like.

One or more adjuvants may also be included with an active composition. Exemplary of useful adjuvants are chelating agents such as ethylenediaminetetraacetic acid (EDTA) that bind calcium ions and assist in passage of medicinal agents through the mucosa and into the blood stream. Another illustrative group of adjuvants are the quaternary nitrogen-containing compounds such as benzalkonium chloride that also assist medicinal agents in passing through the mucosa and into the blood stream.

In addition to the above ingredients, there may also be incorporated other additives selected from among the various pharmaceutically acceptable additives available to the those skilled in the art for the purpose of obtaining desirable processing and physical qualities including enhancement of the dispersibility and stability of the active ingredient. Such additives which can be used in addition to the active ingredient include the following substances: stabilizers/preservatives, e.g., parahydroxybenzoic acid alkyl esters, antioxidants, antifungal agents, and the like; coloring agents, e.g., aluminum lake, titanium dioxide, and the like; excipients/disintegration modulating agents, e.g., magnesium silicate, silicic acid anhydride, aluminum silicate, calcium carbonate, magnesium aluminum metasilicate, calcium hydrogen phosphate, and the like; stearic acid and its salts; palmitic acid; talc; and other substances known as emulsifiers, dispersants, binders, thickeners and the like.

### POLYCARBOPHIL COMPONENT

Several types of materials are suitable for forming the polycarbophil type composition component. The polymer contains a plurality of a repeating unit of which at least about 80 percent contain at least one carboxyl functionality and about 0.05 to about 1.5 percent cross-linking agent substantially free from polyalkenyl polyether, with the percentages being based upon the weights of the unpolymerized repeating unit and cross-linking agent, respectively. In more preferred practice, at least about 90 percent of the repeating units contain at least one carboxyl functionality, and in still more preferred practice, at least 95 percent of those repeating units contain at least one carboxyl functionality. Most preferably, this material is a reaction product of the polymerization of only a carboxyl-functional monomer and a cross-linking agent. Also in more preferred practice, this component contains about 0.1 to about 1 percent by weight of polymerized cross-linking agent. When present as calcium polycarbophil, the material also contains from 5% to 25%, preferably 18% to 22% calcium as a calcium salt of the polymer acid. Certain species of this type of polymer is commercially available under the generic name "calcium polycarbophil".

A polycarbophil type composition polymer useful herein may thus be defined as a reaction product of the copolymerization of at least 80 weight percent monoethylenically unsaturated carboxy-functional monomer and about 0.05 to about 1.5 weight percent of a cross-linking agent free of polyalkenyl polyether and, if present as calcium polycarbophil, 18-22% of calcium. The remaining monomers that may be present to constitute 100 percent by weight of the monomers.

In addition to the above two ingredients, the polycarbophil type polymer may also include polymerized monoethylenically unsaturated repeating units such as C1-C6 alkyl esters of one or more of the above-described acids such as hexyl acrylate, butyl methacrylate and methyl crotonate; hydroxyalkylene-functional esters of the above-described acids that contain a per molecule average of 1 to about 4 oxyalkylene groups containing 2-3 carbon atoms such as hydroxyethyl methacrylate, hydroxypropyl acrylate and tetraethylene glycol monoacrylate; methacrylamide, acrylamide and their C1-C4 mono- and di-alkyl derivatives such as N-methyl acrylamide, N-butyl methacrylamide and N,N-dimethyl acrylamide; styrene; and the like as are known in the art as being copolymerizable with the above described carboxyl functionality-containing monomers and cross-linking agents. The polymers most preferably are prepared from only the monoethylenically unsaturated carboxy-functional monomer and the cross-linking agent.

The (calcium) polycarbophil type composition useful herein may be prepared by conventional free radical polymerization techniques utilizing initiators such as benzoyl peroxide, azobisisobutyronitrile, and the like, are polymerized in an aqueous medium, and are not agglomerated by steam action. A particularly preferred polycarbophil component that is commercially available is that material sold under the designation calcium polycarbophil by the B. F. Goodrich Co. of Cleveland, Ohio. The United States Pharmacopeia, 1990 edition, United States Pharmacopeial Convention, Inc., Rockville, MD., at page 218, indicates that calcium polycarbophil is a calcium salt of polyacrylic acid cross-linked with divinyl glycol that has a calcium content of not less than 18% and not more than 22% and absorbs not less than 35 grams of sodium bicarbonate solution per one gram of the powder in the test under Absorbing power.

The polycarbophil component must be present in the form of its calcium salt when the compositions do not contain alginic acid. The calcium should be present in an amount from about 5 to about 25 percent and preferably from about 18 to about 22 percent, based on the weight of polycarbophil. Most preferably, the calcium cation is originally present as the salt of the polycarbophil type composition. However, it may also be otherwise introduced as a calcium ion-containing compound, such as calcium chloride, calcium gluconate. calcium hydroxide, or the like.

The interaction of the polycarbophil type composition with the water, if so desired, should be at a pH of from about 1 to about 13, preferably at a pH from about 2.0 to about 9.5. Interaction at such a pH will assure that the polycarbophil type composition and water form the desired structure.

The polycarbophil type component may be interacted with the water in any suitable means. Water may be mixed with other cosolvents e.g. glycerol, propylene glycol, or polyethylene glycols, in varying proportion to affect the formation of the polymeric hydrogel.

### INCORPORATION OF ACTIVE AGENT

A composition of this invention is an intimate mixture of the polymeric matrix and the active composition and includes mixtures formed at the time of administration by mixing various components. The phrase "intimate mixture" is used herein to mean that the components of the composition are mixed substantially uniformly so that none of those components is localized. A minor amount of agitation immediately prior to use may be required for some liquid compositions of this invention to achieve an intimately mixed state when used. In two part preparations, the polymer phase and the liquid phases are mixed just before application. Once the two phases are mixed, the composition is such as defined above. Alternatively, the polymer composition in nonpolar solvents and cosolvents may be introduced into or onto, for example, the body, either parenterally or locally, and the hydrogel formation completed by the water present in the body fluid at the site of application.

Without the use of water at all, or prior to interacting the calcium polycarbophil with the water, the active composition as described previously may be incorporated into the polycarbophil type composition. The incorporation may be through dissolution, or dispersion of the active composition in a matrix of the calcium polycarbophil. The amount of polycarbophil type composition will affect the consistency of the final product. Accordingly, the final product may vary from a water-like consistency to that of a solid dry powder, or directly compressed tablet. The polycarbophil type composition may be introduced into the reaction as a solid, in an aqueous or a mixed solvent system, or in a nonaqueous solvent or carrier. All percentages expressed in this application are by weight unless stated otherwise.

The interaction of the calcium polycarbophil with the active agent results in the formation of a polymeric matrix structure which then acts to control the diffusion or other transport of the active composition within and from the matrix itself. If water is also present, the resultant composition is a complex hydrogel.

### THE ALGINIC ACID COMPONENT

Alginic acid is a purified polysaccharide, linear polymer which is extracted from seaweed and is available in many forms, especially as a calcium, potassium, or sodium salt, or as propylene glycol alginate. Preferably, the sodium salt is employed in the present invention.

The interaction of the polycarbophil type composition component with the alginic acid or alginate must occur in the presence of a divalent cation. Preferably, the divalent cation is calcium, although it may also be magnesium. In certain instances divalent iron, copper, or chromium may also be used. The divalent cation should be present in an amount from about 5 to about 25 percent and preferably from about 18 to about 22 percent, based on the weight of polycarbophil. Most preferably, the calcium cation is originally present as the salt of the polycarbophil type composition. However, it may also be initially present as the salt of the alginic acid or may otherwise introduced as a calcium ion-containing compound, such as calcium chloride, calcium gluconate. calcium hydroxide, or the like.

### INTERACTING THE POLYCARBOPHIL COMPONENT AND ALGINIC ACID

For the bioadhesive compositions of the present invention, containing alginic acid, preferably, the active composition is incorporated into the alginate prior to the interaction with the polycarbophil type composition component.

The interaction of the polycarbophil type composition with the alginic acid or alginate should be at a pH of from about 3 to about 10, preferably at a pH from about 6.5 to about 7.5. Interaction at such a pH will assure that the polycarbophil type composition and alginic acid components form the desired complex. It is believed that the complex is formed through a bridging of carboxyl groups of the two polymeric components that occurs through the divalent cation.

The polycarbophil type component may be interacted with the alginic acid in any suitable means. Preferably, the alginic acid is present in the form of its sodium salt, while the polycarbophil type composition is present in the form of its calcium salt. Most preferably, the polycarbophil type composition component is that sold under the designation calcium polycarbophil. The commercially available calcium polycarbophil is not itself a bioadhesive.

Prior to interacting the two components, an active composition as described previously may be incorporated into one or both of the polycarbophil type composition and the alginate. The incorporation may be through dissolution, or dispersion of the active composition in a matrix of the alginate, for example, as a solid or a semi-solid or the alginate may be in an agueous or mixed solvent system.

The alginate component may be employed in an amount from about 0.1 to about 99.9 percent, based upon the total weight of the composition. The amount of polycarbophil type composition will affect the consistency of the final product. Accordingly, the final product may vary from a water-like consistency to that of a solid dry powder. Likewise, the amount of calcium polycarbophil type composition may vary from about 0.1 to about 90.0 percent, based upon the total weight of the composition. The polycarbophil type composition may be introduced into the reaction as a solid or in an aqueous or a mixed solvent system. All percentages expressed in this application are by weight unless stated otherwise.

The interaction of the two components in the presence of water or other polar solvent and in the presence of a divalent cation, especially calcium, results in the formation of a complex which may be described as membrane type matrix, specifically at the interface of the two polymers. The resulting matrix structure then acts to control the diffusion or other transport of the active composition within and from the matrix itself.

The desired level of controlled or sustained release will vary, depending upon the ratio of the components employed, the particular active composition, the method of incorporation, the order of mixing of the components, and the like. Additional additives may also be present which may modify the characteristics of the matrix and its release properties.

### GENERAL FORMULATION CONSIDERATIONS

When the composition of the present invention contains an active agent that is a cosmetic agent, household agent, agricultural agent, or nutritional agent, the composition of the present invention will contain an effective amount of such an active agent which is that amount which in a composition of this invention provides a sufficient amount of the active composition to provide a measurable or discernable amount of the desired activity of the active composition.

The active composition, when a medicinal agent, is present in the compositions of this invention in an amount that is sufficient to prevent, cure and/or treat a condition for a desired period of time for which the composition of this invention is to be administered, and such an amount is referred to herein as "an effective amount". As is well known, particularly in the medicinal arts, effective amounts of medicinal agents vary with the particular agent employed, the condition being treated and the rate at which the composition containing the medicinal agent is eliminated from the body, as well as varying with the animal in which it is used, and the body weight of that animal. Consequently, effective amounts of active compositions may not be defined for each agent. Thus, an effective amount is that amount which in a composition of this invention provides a sufficient amount of the active composition to provide the requisite activity of active composition in or on the body of the treated animal for the desired period of time, and is typically less than that amount usually used.

Inasmuch as amounts of particular active compositions in the blood stream that are suitable for treating particular conditions are generally known, as are suitable amounts of active compositions used in cosmetics, it is a relatively easy laboratory task to formulate a series of controlled release compositions of this invention containing a range of such active compositions to determine the effective amount of such an active composition for a particular composition of this invention. While the effective amount for all active compositions cannot be stated, typical compositions of this invention may contain about one microgram to about one gram of active composition per dose administered. More preferably, a composition of this invention may contain about one microgram to about 400 milligrams per dose.

In typical practice, the ratio by weight of the polymeric complex to the active composition in the composition is about 200,000:1 to about 1:100. In preferred practice, however, the weight ratio of polymeric complex to active composition is about 100,000:1 to about 10:1. Those weight ratios are determined using dry ingredients.

In addition to the active composition and polymeric complex, the compositions of this invention may also contain diluents, such as pharmaceutically acceptable diluents and/or one or more materials present as a medicinally inert matrix.

The controlled release compositions of the present invention will include an active agent in an amount within the range of from about 0.0001 to about 65% by weight, preferably in an amount within the range of from more than 0.100 to about 65% by weight of the composition and more typically in the range of about 1 to about 35 %. The (calcium) polycarbophil polymer will be present in an amount within the range of from about 0.1 to about 99.9%, and preferably from about 0.25% to about 90% by weight of the composition, and most preferably from about 0.25 to about 50%; and the auxiliary excipients may be present in an amount within the range of from about 0.01 to about 99%, and preferably from about 0.25 to about 50% by weight of the composition. Water, if present, will typically be present in an amount of about 0.1 to about 200% and preferably from about 0.5 to about 100%, and most preferably from about 10 to about 100 %, based upon the weight of the (calcium) polycarbophil. The amount of water added may vary depending upon whether other excipients are present. The water content as described herein is the amount added to initiate the formation of a hydrogel. Once the gel is formed, the water may be removed to obtain a dry powder, granules or a matrix, or more water may be added to obtain the desired consistency. Alternatively, the controlled release compositions of the present invention may be formed directly with calcium polycarbophil and an active agent, with or without other excipients, by direct procedures, such as direct compression, direct extrusion, direct blending and direct molding.

The sustained release matrix, if containing a pharmaceutical agent, will optionally include additional edible non-toxic ingredients as conventionally employed in medicinal dosage forms. Thus, the compositions of the invention may optionally include one or more excipients in an amount within the range of from about 0.1% to about 99% by weight and preferably from about 1% to about 95% by weight, such as lactose, sugar, corn starch, modified corn starch, mannitol, sorbitol, artificial sweeteners, and inorganic salts such as calcium carbonate. Other conventional ingredients which may optionally be present include preservatives, stabilizers, plasticizers, cosolvents, anti-adherents or silica flow conditioners or glidants, such as Syloid brand silicon dioxide as well as FD&C colors.

The pharmaceutical and cosmetic compositions of this invention are substantially non-toxic to the animals in which or on which they are placed, aside from any toxicity associated with the active composition alone. Thus, when contacted with and adhered to skin or mucosa, the compositions cause no apparent whitening or blistering effects due to the compositions. In addition, adverse immunologic effects from the use of compositions of this invention in animals should not be present.

The phrases "pharmaceutically acceptable", "physiologically tolerable" and "medicinally inert" are used herein to mean that the material so described may be used for treatments in or on humans or other mammals without causing ill effects, such as toxicity, blistering or whitening of mucosa or skin tissues, and that those materials are not themselves active compositions or bioadhesives, as those terms are used herein.

### DOSAGE FORMS

The invention is broadly applicable to making a wide variety of dosage forms such as tablets, including but not limited to, antacid tablets, cough medicine tablets, sore throat tablets, breath freshener tablets, vitamin tablets, calcium tablets, dietary supplement and nutrient tablets, laxative tablets, cold tablets, analgesic tablets, anti-diarrhea tablets, reducing tablets, pain reliever tablets, sleeping tablets, and many prescription and non-prescription drug and pharmaceutical tablets.

For example, a composition may be an intimate mixture of the polymeric system and active composition in either dry form, as a semi-solid or as a liquid suspension. The composition may also be provided as a three-dimensional structure such as a capsule, a capsule aggregate, a film or laminate. When provided as a three-dimensional structure, the active composition is contained in an inert matrix.

Illustrative of an intimate mixture of dry composition components is an admixed powder formed from comminuted polymeric complex particles having a size sufficient to pass through a 20 mesh sieve screen (841 µm) and be retained on a 200 mesh sieve screen (74 µm) (U.S. Standard Sieve Series) admixed with similarly or smaller sized particles of an active composition such as chlorothiazide. (Hereinafter, when particles are sized to pass through one screen and be retained on a second screen, as above, the size of the passing screen mesh will be written first, followed by a virgule, "/", and then the size of the retaining screen mesh. Thus, the above passing and retaining screen mesh sizes are written "20/200".) The mixture may be provided for treatment in tablet form or within a gelatin capsule and ingested for treatment.

The size of the polymeric complex particles has some effect upon the compositions of this invention. It is apparent that the polymeric complex particles should not be so large that the composition cannot be administered without undue difficulty. For example, if the composition is to be swallowed, the polymeric complex particles must be sized to permit passage of the composition to the stomach without impeding passage of subsequently ingested foods or liquids thereto. Similarly, when the composition is to be used in the eye, finely comminuted polymeric complex particles, e.g. sized to pass through a 100 mesh sieve screen (U.S. Standard Sieve Series) sieve opening: 150 µm, are utilized so that visual impairment of the treated animal is minimized.

Typically, at the maximum, a useful polymeric complex is sized to pass through a sieve screen having a 10 mesh (U.S. Standard Sieve Series); i.e., a 2000 µm opening. Preferably, the polymeric complex particles are sized to pass through a 30 mesh sieve screen (595 µm) (U.S. Standard Sieve Series). Particles having a relatively small size swell more rapidly than do particles having a relatively large size, and thus, a relatively small size is preferred for the particles.

In another embodiment, the polymeric complex is swollen in an aqueous medium containing the active composition, and the active composition is sorbed (absorbed or adsorbed) into or onto the swollen polymeric complex particles. After drying, the composition so prepared is provided for treatment as described above. The word "dry" is used herein in relation to a polymeric complex to mean that the polymeric complex does not adhere when touched with a finger within a rubber glove, and is substantially unswollen.

A composition of this invention may also be provided in two parts, a polymer phase containing (calcium) polycarbophil, optionally with other excipients, and a liquid phase containing water and optionally cosolvents. One or more active agents, such as medicinal agents, may be incorporated in either one phase or both of the phases. When the polymer and liquid parts are mixed, "polymerization" ensues.

A composition of this invention may also be provided as a suspension of (calcium) polycarbophil and optionally other excipients in nonpolar solvents e.g. vegetable oils and synthetic oils, which when it comes into contact with, for example, body fluid (water), as through parenteral injection, implantation or local application, will "polymerize" to form a complex hydrogel matrix in situ and provide a controlled release delivery system. Such a system is useful for parenteral and local applications.

The dosage form can be packaged in unit dose blister packs, pouches in a carton, vials with screw or flip-top lids, bottles with screw or flip-top lids, or any other convenient package form.

The compositions of the present invention also may be employed with suppositories for rectal or vaginal administration. In such embodiments, the polymeric complex may be coated on the surface of an active composition-containing suppository or it may be dispersed therein.

The controlled release composition containing the alginic acid adheres to the skin or to mucus membranes (mucosa) in the presence of sufficient water to swell the polymeric complex.

### METHOD OF TREATMENT

For pharmaceutical compositions, a controlled release method of treatment is also contemplated. According to this method, a controlled release composition of this invention is provided. A composition of the present invention may be placed into the oral, buccal, or gingival area. Alternatively, in the method of controlled release treatment a controlled release composition containing an effective amount of active composition per dose is provided, as described before and may be initially present or may be formed in situ. An area of skin or mucus membrane to be treated is contacted with the provided composition. Each of the beforedescribed compositions may be administered in accordance with this method.

For purposes of in situ formation of the pharmaceutical complexes of the present invention, it is necessary to deliver the (calcium) polycarbophil as a relatively fluid composition, as by parenteral injection. In such instances, the use of water or other liquids capable of initiating hydrogen bonding of the (calcium) polycarbophil should be avoided. It is generally preferred to use as a carrier for the (calcium) polycarbophil a liquid which is a nonpolar solvent, such as an animal or vegetable oil, or a synthetic oil, such as a silicone. The carrier for such purposes then is usually hydrophobic. For certain applications, however, it may be possible to employ glycerine, propylene glycol, polyethylene glycols or other mono or polyhydric alcohols as a carrier for the (calcium) polycarbophil. The (calcium) polycarbophil may be suspended in the appropriate carrier as small micro-particles capable of being parenterally injected or otherwise introduced into a subject.

A controlled release method of treatment is also contemplated. According to this method, a controlled release composition of this invention is provided. An area of skin or of mucus membrane to be treated is contacted with that composition, with the contacting being carried out in the presence of sufficient water to swell the polymeric complex. The composition adheres to the area contacted, releasing the active composition at a controlled rate, and causing the active composition to be sorbed at least at the vicinity of the contacted area.

Upon contact with mucus which is excreted by the lining of the buccal cavity, the polymeric complex hydrates thus adhering the buccal dosage form to the lining.

The compositions of this invention may provide the intimate contact between the active composition and the mucosa that is preferred for high molecular weight active compositions such as proteins and hormones. These compositions, through their bioadhesion, maintain the intimate contact for extended periods of time to thereby increase the relative concentration of the active composition in the vicinity of that contact.

Each of the beforedescribed compositions may be administered in accordance with this method.

The compositions of this invention may be administered by several means to provide the desired contact between the skin or mucus membrane and the composition. For example, where the active composition is a sun screen, the composition may be applied to the skin by rubbing the composition over the skin area to be treated. Where the conjunctival mucosa are to be contacted, the aqueous composition described above may be instilled into the precorneal pockets of the eyes. Where the buccal, nasal, anal and/or vaginal mucosa are to be contacted, the composition may be applied by hand, forceps or other suitable instrument. Where the mucosa of the stomach and/or intestines are to be contacted, the composition is typically swallowed, or implanted surgically, and contact with the mucus membrane is achieved by the contraction of the stomach or intestines and/or by the carrying action provided by passage of gastric fluids therethrough.

The composition is left in place (contact maintained) for a time sufficient for the active composition to be released over a controlled period, and thereby provide its medicinal or cosmetic function to the treated animal. In most circumstances, some unused, still active, controlled release composition and the remainder of the composition administered are eliminated from the body by a natural bodily mechanism, such as by dispersion or erosion caused by an aqueous body fluid such as saliva, tears, gastric fluid or vaginal secretions. In other instances, such as where the active composition is contained in a medicinally inert matrix such as a ethylene-vinyl acetate copolymer or cross-linked elastomer that is ingested, the flushing action provided by the flow of gastric fluids and stomach contractions ultimately results in excretion in the feces of any unused composition and of the remainder of the administered composition. The desired level of controlled or sustained release will vary, depending upon the ratio of the components employed, the physical state of the components of the particular active composition, the method of incorporation, the order of mixing of the components, and the like. Additional additives may also be present which may modify the characteristics of the matrix and its release properties.

### TREATMENT OF XEROSTOMIA

For treatment of dry mouth, or xerostomia, the polycarbophil is formed into a wafer, disc, or other shape suitable for insertion into the mouth. Most preferably, the shape of the polycarbophil follows the natural contour of the mouth and can be made by any suitable technique, such as molding, casting, and the like.

Preferably the polycarbophil, after formation into a suitable shape, is hydrated. Generally the shaped polycarbophil should contain from about 1 to about 10 ml of water, preferably from about 2 to about 5 ml. The mere presence of the polycarbophil in the mouth, however, even if not initially hydrated before insertion, may stimulate sufficient salivation to both hydrate the polycarbophil and to counteract the dry mouth condition.

In use, all that is necessary is for the shaped polycarbophil to be inserted into the mouth and maintained within the mouth for a sufficient period of time to alleviate the feeling of dryness in the mouth and to cause hydration of the mouth. While so present the hydrated polycarbophil acts to humidify the mouth, while in some instances also stimulating saliva production. Of particular advantage is the fact that the polycarbophil is nontoxic, so that accidental swallowing of the polycarbophil should result in no ill effect. Of course, shaped polycarbophil also containing active medicaments, lubricants, flavorants, and the like may also achieve the desired effect of treating the dry mouth condition.

The present invention is further illustrated by the following examples which are not intended to limit the full scope and content of the present invention as described herein and in the appended claims.

### EXAMPLE 1

This example demonstrates the manufacture of a composition of the present invention which is a controlled release oral tablet dose form containing pilocarpine as an active agent. Calcium Polycarbophil, USP (Carbopol EX-83 Resin, Lot No. Z139117, B.F. Goodrich) is used in this example. Other grades of varying particle size and surface area materials of this resin may be used to obtain other desirable properties.

Sixty parts of calcium polycarbophil, USP, ten parts of Carbopol® 934-P, ten parts of directly compressible sugar and seventeen parts of directly compressible dicalcium phosphate dihydrate with two parts of pilocarpine hydrochloride, USP, are mixed together for ten minutes. One part of magnesium stearate, USP, is blended in for about two minutes. The blended mass is compressed directly on a rotary tablet machine to obtain tablets of an average weight of two hundred milligrams.

### Testing

The tablets are tested for dissolution of pilocarpine using a suitable apparatus. It is noted that the tablets that were submerged in water became hard and rubbery in consistency. Pilocarpine is released at a near zero-order rate over a time period of twelve hours.

It is very clear from this experiment that this formulation can serve as a controlled release application for a variety of products. The main reason for the controlled activity is the tendency of calcium polycarbophil to form a rubbery, spongy hydrogel material when mixed with water. It appears that water causes internal structurization and "polymerization" by hydrogen bonding.

### EXAMPLE 2

This example demonstrates the manufacture of a composition of the present invention which is a controlled release candy composition using calcium polycarbophil and a breath freshener. Calcium Polycarbophil, USP (Carbopol EX-83 Resin, Lot No. Z139117, B.F. Goodrich) is used in this example. Other grades of varying particle size and surface area materials of this resin may be used to obtain other desirable properties.

Sixty grams of calcium polycarbophil is mixed with five hundred milligrams of Peppermint Oil, NF. In a separate container, twenty grams of Mannitol, USP are mixed with twenty grams of sodium alginate (Keltone HV, Kelco Co). Additionally, five hundred milligrams of Peppermint Oil, NF are added to this mixture and mixed well.

About forty grams of Purified Water, USP are added to the calcium polycarbophil mixture prepared above and the preparation is mixed well rapidly. The preparation is allowed to gel for about 45 seconds, at which time, the mannitol and sodium alginate mixture prepared above is added and the preparation is mixed well to obtain uniformly wet spongy mass. The mass is then rolled into small spaghetti type rolls and small pieces of about 200 milligrams weight are cut. The pieces are rolled by hand into small balls of about 1/8-1/4" 3,2-6,35 mm diameter. The balls so produced are dried at 37°C for twenty four hours.

### Testing

The dried balls were observed to be of very hard marble type candies. The ball was noted to disintegrate in water maintained at 37°C over 2-6 hours under mild agitation. The ball was observed to dissolve slowly in the mouth over two to six hours and Peppermint Oil was noted to release over this time period. The ball (candies) was non-gummy, non-sticky and provided a pleasant, mouth freshening over the time period. The candy did not break but dissolved slowly even when it was swished around in mouth constantly.

### EXAMPLE 3

This example demonstrates the manufacture of a composition of present invention which is supplied as a "two component system" which is suitable as a controlled release pour on dressing for burn treatment. Calcium polycarbophil, USP (Carbopol EX-788 Resin, B.F. Goodrich) is used in this example.

Fifty grams of Calcium polycarbophil is sterilized by dry heat sterilization method. The sterile powder is mixed with five grams of sterile xanthan gum (Keltrol-T, Kelco Co.). The dry powder is packaged into suitable glass containers as "Solid Phase 1". In a separate container, 40 milliliters of sterile solution containing 1% silver nitrate is prepared. To prepare the "pour-on" burn dressing, the silver nitrate solution is added to the calcium polycarbophil/Keltrol mixture. The suspension is mixed well to disperse the material and to initiate polymerization. After sixty seconds, the preparation begins to thicken. It is suitable to be slowly poured over the affected area to form an on-site wound dressing.

In the laboratory trial, the dressing was poured onto a flat glass plate. The preparation solidified and appeared dry within less than two minutes and became like a spongy woven fabric. Small pieces of this preparation were cut and submerged in 100 ml. of deionized water (having an electrical resistance of 18 mega ohms) and maintained at 37°C. The samples were gently shaken, continuously. The water was removed and a fresh quantity of water added at specified sampling time intervals. The water samples were tested for the presence and release of silver nitrate. The pieces were found to release silver nitrate at a fairly constant rate over a 72 hour time period. After about 96 hours, silver nitrate was completely released from the matrix. In a separate experiment, the preparation was poured onto a subject's hand. The preparation solidified within a few seconds into a spongy mass. The preparation stayed on without causing any irritation, burning or discomfort and peeled off very easily without causing any pain.

It is very clear from this experiment that this formulation can serve as controlled release application for local and bolus type of applications.

### EXAMPLE 4

Eighty parts of calcium polycarbophil are mixed with nine parts each of mannitol and dicalcium phosphate dihydrate. To the blended mass, one part of light mineral oil, USP is added. The mass is mixed well. One part of magnesium stearate is added to the above and the whole mass is mixed for two minutes. A small flat disc of 3/4" X 2" 19 X 50,1 mm size is formed by compressing the powder under pressure. The resultant compact upon submersion in water forms a spongy rubbery flat article. It is found that this article is suitable for keeping mouth lubricated. The flat hydrated discs are found suitable for moisturizing and lubricating dry mouth and are suitable for keeping in the mouth, for extended periods, especially at bedtime.

It is clear from this experiment that this formulation can serve as a device to overcome dry mouth problems and provide significant relief to xerostomiacs, particularly at night. Similar devices can be produced for a variety of applications.

### EXAMPLE 5

This example demonstrates the manufacture of a composition of the present invention in which benzocaine is incorporated for the purposes of achieving controlled release. Five-hundred milligrams of sodium alginate (Algin HV from Kelco) are mixed with eighty milligrams of benzocaine, USP. The resultant mass is granulated using 10% glycerol solution in purified water, USP, as the binding solution. Propylene glycol solution may also be employed in place of the glycerol. The resultant granulated mass is further mixed with five-hundred milligrams of calcium polycarbophil, USP. The mass is further granulated with the aid of purified water as a binder. Corn starch could also be employed at this granulation stage. An additional twenty milligrams of benzocaine are added to the mass with mixing. The resulting product is then conventionally formed into thin discs or wafers suitable for local administration.

The resulting discs are formulated of varying sizes, making them suitable for buccal, gingival or oral administration. The ratio of the polymeric complex material of the present invention to the weight of the active composition is approximately 10:1).

### EXAMPLE 6

This example demonstrates the manufacture of a composition of the present invention which is a controlled release candy composition using calcium polycarbophil and breath freshener. Calcium Polycarbophil, USP (Carbopol EX-83 Resin, Lot No. Z139117, B.F. Goodrich) is used in this example. Other grades of varying particle size and surface area materials of this resin may be used to obtain other desirable properties.

Sixty grams of calcium polycarbophil is mixed with five hundred milligrams of Peppermint Oil, NF. In a separate container, twenty grams of Mannitol, USP are mixed with twenty grams of sodium alginate (Keltone HV, Kelco Co). Additionally, five hundred milligrams of Peppermint Oil, NF are added to this mixture and mixed well.

About forty grams of Purified Water, USP are added to the calcium polycarbophil mixture prepared above and the preparation is mixed well rapidly. The preparation is allowed to gel for about 45 seconds, at which time, the mannitol and sodium alginate mixture prepared above is added and the preparation is mixed well to obtain uniformly wet spongy mass. The mass is then rolled into small spaghetti type rolls and small pieces of about 200 milligrams weight are cut. The pieces are rolled by hand into small balls of about 1/8-1/4" 3,2-6,35 mm diameter. The balls so produced are dried at 37°C for twenty four hours.

### Testing

The dried balls were observed to be of very hard marble type candies. The ball was noted to disintegrate in water maintained at 37°C over 2-6 hours under mild agitation. The ball was observed to dissolve slowly in the mouth over two to six hours and Peppermint Oil was noted to release over this time period. The ball (candies) was non-gummy, non-sticky and provided a pleasant, mouth freshening over the time period. The candy did not break but dissolved slowly even when it was swished around in mouth constantly.

It is very clear from this experiment that this formulation can serve as a controlled release application for a variety of products. The main reason for the controlled activity is the tendency of calcium polycarbophil to form a rubbery, spongy hydrogel material when mixed with small and limited quantities of water. It appears that water causes internal structurization and "polymerization" by hydrogen bonding. The incorporation of sodium alginate and/or mannitol enables one to achieve the desired breakdown and bonding of the mass. The addition of mannitol is also for achieving the desired taste and cooling effect when the candy is sucked.

### EXAMPLE 7

This example demonstrates the manufacture of a composition of present invention which is supplied as a "two component system" which is suitable as a controlled release pour on dressing for burn treatment. Calcium polycarbophil, USP (Carbopol EX-788 Resin, B.F. Goodrich) is used in this example.

Fifty grams of Calcium polycarbophil is sterilized by dry heat sterilization method. The sterile powder is mixed with five grams of sterile xanthan gum (Keltrol-T, Kelco Co.). The dry powder is packaged into suitable glass containers as "Solid Phase 1". In a separate container, 40 milliliters of sterile solution containing 1% silver nitrate is prepared. To prepare the "pour-on" burn dressing, the silver nitrate solution is added to the calcium polycarbophil/Keltrol mixture. The suspension is mixed well to disperse the material and to initiate polymerization. After sixty seconds, the preparation begins to thicken. It is suitable to be slowly poured over the affected area to form an on-site wound dressing.

In the laboratory trial, the dressing was poured onto a flat glass plate. The preparation solidified and appeared dry within less than two minutes and became like a spongy woven fabric. Small pieces of this preparation were cut and submerged in 100 ml. of deionized water (having an electrical resistance of 18 mega ohms) and maintained at 37°C. The samples were gently shaken, continuously. The water was removed and a fresh quantity of water added at specified sampling time intervals. The water samples were tested for the presence and release of silver nitrate. The pieces were found to release silver nitrate at a fairly constant rate over a 72 hour time period. After about 96 hours, silver nitrate was completely released from the matrix. In a separate experiment, the preparation was poured onto a subject's hand. The preparation solidified within a few seconds into a spongy mass. The preparation stayed on without causing any irritation, burning or discomfort and peeled off very easily without causing any pain.

It is very clear from this experiment that this formulation can serve as controlled release application for local and bolus type of applications.

This invention has been described in terms of specific embodiments set forth in detail, but it should be understood that these are by way of illustration only and that the invention is not necessarily limited thereto.

## Claims (Claims for the following Contracting State(s): AT, BE, CH, LI, DE, DK, FR, GB, IT, LU, NL, SE)

1. A polymeric complex composition comprising a reaction complex formed by the interaction of (1) a calcium polycarbophil component which is a water-swellable, but water-insoluble, fibrous, cross-linked carboxy-functional polymer, said polymer containing (a) a plurality of repeating units of which at least 80 percent contain at least one carboxyl funcationality, and (b) 0.05 to 1.5 percent cross-linking agent free from polyalkenyl polyether, said percentages being based upon the weights of unpolymerized repeating unit and cross-linking agent, respectively with (2) alginic acid or a salt thereof, said interaction being performed in the presence of a divalent cation.

2. The composition according to claim 1 wherein the divalent cation is calcium.

3. The composition according to claim 1 or 2 wherein said polymeric complex composition further comprises one or more adjuvants or one or more pharmaceutical stabilizers.

4. The composition according to any preceding claim wherein the composition contains one or more further components selected from polar and nonpolar cosolvents, carbohydrates, hydrocolloids, chelating agents, quaternary nitrogen-containing compounds, preservatives, antioxidants, excipients, disintegration modulating agents, emulsifiers, dispersants, binders, thickeners, artificial sweeteners, antifungal agents, coloring agents, flavors, and breath fresheners.

5. The composition according to any preceding claim wherein said polymeric complex composition further comprises one or more active agents.

6. The composition according to claim 5, wherein the active agent is selected from a household agent, a cosmetic agent, a nutritional agent, a medicament and an agricultural agent.

7. The composition according to claim 5 wherein said polymeric complex is in the form of comminuted particles capable of passing through the screen of a sieve having a 100 mesh size, U.S. Standard Sieve Series, (sieve opening: 150µm) and said active agent is present as a saturated solute in a physiologically acceptable aqueous carrier.

8. The composition according to claim 5 wherein said active agent is contained in a medicinally inert matrix in the form of a three-dimensional structure having at least one surface portion, and said polymeric complex is disposed on at least said one surface portion.

9. The composition according to claim 8 wherein said three-dimensional structure is a film.

10. The composition according to claim 5 wherein the ratio by weight of said polymeric complex to said active agent is 200,000:1 to 1:100.

11. A polymeric complex composition comprising (1) a calcium polycarbophil component which is a water-swellable, but water-insoluble, fibrous, cross-linked carboxy-functional polymer, said polymer containing (a) a plurality of repeating units of which at least 80 percent contain at least one carboxyl functionality, and (b) 0.05 to 1.5 percent cross-linking agent free from polyalkenyl polyether, said percentages being based upon the weights of unpolymerized repeating unit and cross-linking agent, respectively with (2) water.

12. The composition according to claim 11 further comprising one or more active agents.

13. The composition according to claim 11 or 12 further comprising one or more adjuvants or one or more pharmaceutical stabilisers.

14. The composition according to claim 11, 12 or 13 wherein the composition contains one or more further components selected from polar and nonpolar cosolvents, carbohydrates, hydrocolloids, chelating agents, quaternary nitrogen-containing compounds, preservatives, antioxidants, excipients, disintegration modulating agents, emulsifiers, dispersants, binders, thickeners, artificial sweeteners, antifungal agents, coloring agents, flavors, and breath fresheners.

15. The composition according to claim 12 wherein the active agent is selected from a household agent, a cosmetic agent, a nutritional agent, a medicament or an agricultural agent.

16. The composition according to claim 11 wherein the calcium is present in an amount from 5 to 25 percent and preferably from 18 to 22 percent, based on the weight of polycarbophil.

17. The composition according to claim 12 wherein the ratio by weight of said polymeric matrix to said active agent is from 200,000:1 to 1:100.

18. A polymeric controlled release system comprising the polymeric complex of any of claims 1 to 17.

19. Use of the composition of according to any of claims 1 to 17 for preparation of a medicament for controlled release therapy.

20. Use of the polymeric controlled release system according to claim 18 for preparation of a medicament for controlled release therapy.

21. Use of calcium polycarbophil in the manufacture of a shaped medical article for treating xerostomia.

22. A composition useful in the treatment of xerostomia comprising an article in a shape suitable for insertion into a mouth of a patient suffering from xerostomia, said article comprising calcium polycarbophil and from 1 to 10ml of water.

23. Use of the composition of claim 22 for the preparation of a medicament to treat xerostomia.

## Claims (Claims for the following Contracting State(s): ES, GR)

1. A polymeric complex composition comprising a reaction complex formed by the interaction of (1) a calcium polycarbophil component which is a water-swellable, but water-insoluble, fibrous, cross-linked carboxy-functional polymer, said polymer containing (a) a plurality of repeating units of which at least 80 percent contain at least one carboxyl funcationality, and (b) 0.05 to 1.5 percent cross-linking agent free from polyalkenyl polyether, said percentages being based upon the weights of unpolymerized repeating unit and cross-linking agent, respectively with (2) alginic acid or a salt thereof, said interaction being performed in the presence of a divalent cation.

2. The composition according to claim 1 wherein the divalent cation is calcium.

3. The composition according to claim 1 or 2 wherein said polymeric complex composition further comprises one or more adjuvants or one or more pharmaceutical stabilizers.

4. The composition according to any preceding claim wherein the composition contains one or more further components selected from polar and nonpolar cosolvents, carbohydrates, hydrocolloids, chelating agents, quaternary nitrogen-containing compounds, preservatives, antioxidants, excipients, disintegration modulating agents, emulsifiers, dispersants, binders, thickeners, artificial sweeteners, antifungal agents, coloring agents, flavors, and breath fresheners.

5. The composition according to any preceding claim wherein said polymeric complex composition further comprises one or more active agents.

6. The composition according to claim 5, wherein the active agent is selected from a household agent, a cosmetic agent, a nutritional agent, and an agricultural agent.

7. The composition according to claim 5 wherein said polymeric complex is in the form of comminuted particles capable of passing through the screen of a sieve having a 100 mesh size, U.S. Standard Sieve Series, (sieve opening: 150µm) and said active agent is present as a saturated solute in a physiologically acceptable aqueous carrier.

8. The composition according to claim 5 wherein said active agent is contained in a medicinally inert matrix in the form of a three-dimensional structure having at least one surface portion, and said polymeric complex is disposed on at least said one surface portion.

9. The composition according to claim 8 wherein said three-dimensional structure is a film.

10. The composition according to claim 5 wherein the ratio by weight of said polymeric complex to said active agent is 200,000:1 to 1:100.

11. A polymeric complex composition comprising (1) a calcium polycarbophil component which is a water-swellable, but water-insoluble, fibrous, cross-linked carboxy-functional polymer, said polymer containing (a) a plurality of repeating units of which at least 80 percent contain at least one carboxyl functionality, and (b) 0.05 to 1.5 percent cross-linking agent free from polyalkenyl polyether, said percentages being based upon the weights of unpolymerized repeating unit and cross-linking agent, respectively with (2) water.

12. The composition according to claim 11 further comprising one or more active agents.

13. The composition according to claim 11 or 12 further comprising one or more adjuvants or one or more pharmaceutical stabilizers.

14. The composition according to claim 11, 12 or 13 wherein the composition contains one or more further components selected from polar and nonpolar cosolvents, carbohydrates, hydrocolloids, chelating agents, quaternary nitrogen-containing compounds, preservatives, antioxidants, excipients, disintegration modulating agents, emulsifiers, dispersants, binders, thickeners, artificial sweeteners, antifungal agents, coloring agents, flavors, and breath fresheners.

15. The composition according to claim 12 wherein the active agent is selected from a household agent, a cosmetic agent, a nutritional agent, and an agricultural agent.

16. The composition according to claim 11 wherein the calcium is present in an amount from 5 to 25 percent and preferably from 18 to 22 percent, based on the weight of polycarbophil.

17. The composition according to claim 12 wherein the ratio by weight of said polymeric matrix to said active agent is from 200,000:1 to 1:100.

18. A polymeric controlled release system comprising the polymeric complex of any of claims 1 to 17.

19. Use of the composition of according to any of claims 1 to 17 for preparation of a medicament for controlled release therapy.

20. Use of the polymeric controlled release system according to claim 18 for preparation of a medicament for controlled release therapy.

21. Use of calcium polycarbophil in the manufacture of a shaped medical article for treating xerostomia.

22. Use of a composition of comprising an article in a shape suitable for insertion into a mouth, said article comprising calcium polycarbophil and from 1 to 10ml of water, for preparation of a medicament to treat xerostomia.

23. A process for preparing a pharmaceutical composition comprising formulating a composition which comprises a medicament and a polymeric complex composition according to any of claims 1-4, 11, 14 or 16.

24. The process according to claim 23 wherein said polymeric complex is in the form of comminuted particles capable of passing through the screen of a sieve having a 100 mesh size, U.S. Standard Sieve Series, (sieve opening: 150µm) and said medicament is present as a saturated solute in a physiologically acceptable aqueous carrier.

25. The process according to claim 23 which affords a composition wherein said medicament is contained in a medicinally inert matrix in the form of a three-dimensional structure having at least one surface portion, and said polymeric complex is disposed on at least said one surface portion.

26. The process according to claim 25 wherein said three-dimensional structure is a film.

27. The process according to claim 5 wherein the ratio by weight of said polymeric complex to said medicament is 200,000:1 to 1:100.

28. A process for preparing a pharmaceutical composition of a polymeric complex composition comprising (1) a calcium polycarbophil component which is a water-swellable, but water-insoluble, fibrous, cross-linked carboxy-functional polymer, said polymer containing (a) a plurality of repeating units of which at least 80 percent contain at least one carboxyl functionality, and (b) 0.05 to 1.5 percent cross-linking agent free from polyalkenyl polyether, said percentages being based upon the weights of unpolymerized repeating unit and cross-linking agent, respectively with (2) water; and a medicament.

29. The process according to claim 28 wherein the pharmaceutical composition contains one or more adjuvants or one or more pharmaceutical stabilizers.

30. The process according to claim 28 or 29 wherein the pharmaceutical composition contains one or more further components selected from polar and nonpolar cosolvents, carbohydrates, hydrocolloids, chelating agents, quaternary nitrogen-containing compounds, preservatives, antioxidants, excipients, disintegration modulating agents, emulsifiers, dispersants, binders, thickeners, artificial sweeteners, antifungal agents, coloring agents, flavors, and breath fresheners.

31. The process according to claim 28 wherein the calcium is present in an amount from 5 to 25 percent and preferably from 18 to 22 percent, based on the weight of polycarbophil.

32. The process according to claim 28 wherein the ratio by weight of said polymeric matrix to said medicament is from 200,000:1 to 1:100.

## Patentansprüche (Patentansprüche für folgende(n) Vertragsstaat(en): AT, BE, CH, LI, DE, DK, FR, GB, IT, LU, NL, SE)

1. Polymerkomplex-Zusammensetzung, umfassend einen Reaktionskomplex, gebildet durch die Wechselwirkung einer Kalziumpolycarbophil-Komponente (1), die ein in Wasser quellbares, jedoch wasserunlösliches, faseriges, vernetztes Polymer mit Carboxyl-Funktionalität ist, wobei das Polymer (a) eine Vielzahl an wiederkehrenden Einheiten, von denen zumindest 80% zumindest eine Carboxyl-Funktionalität enthalten, und (b) 0,05-1,5% Vernetzer frei von Polyalkenylpolyether enthält, wobei die Prozentangaben auf das Gewicht der wiederkehrenden, nicht polymerisierten Einheiten bzw. des Vernetzers bezogen sind, mit (2) Alginsäure oder einem Salz davon, wobei die Wechselwirkung in Gegenwart eines zweiwertigen Kations erfolgt.

2. Zusammensetzung nach Anspruch 1, worin das zweiwertige Kation Kalzium ist.

3. Zusammensetzung nach Anspruch 1 oder 2, worin die Polymerkomplex-Zusammensetzung weiters ein oder mehrere Adjuvantien oder einen oder mehrere pharmazeutische Stabilisatoren umfaßt.

4. Zusammensetzung nach einem der vorhergehenden Ansprüche, worin die Zusammensetzung eine oder mehrere Komponenten enthält, die aus polaren und apolaren Co-Lösungsmitteln, Kohlenhydraten, Hydrokolloiden, Chelatbildnern, quaternären stickstoffhältigen Verbindungen, Konservierungsmitteln, Antioxidantien, Exzipienten, Zerfallsmodulatoren, Emulgatoren, Dispergatoren, Bindemitteln, Verdickern, künstlichen Süßstoffen, pilzhemmenden Mitteln, Farbstoffen, Geschmacksstoffen und Atemerfrischern ausgewählt sind.

5. Zusammensetzung nach einem der vorhergehenden Ansprüche, worin die Polymerkomplex-Zusammensetzung weiters eine oder mehrere Aktivsubstanzen umfaßt.

6. Zusammensetzung nach Anspruch 5, worin die Aktivsubstanz aus Haushaltsmitteln, Kosmetika, Nährstoffen, Medikamenten und landwirtschaftlichen Mitteln ausgewählt ist.

7. Zusammensetzung nach Anspruch 5, worin der Polymerkomplex in Form von zerkleinerten Teilchen vorliegt, die ein Sieb mit einer Größe von 100 Mesh (US Standard Sieve Series, Sieböffnung: 150 µm) passieren können, und die Aktivsubstanz als gesättigter gelöster Stoff in einem physiologisch annehmbaren, wäßrigen Träger vorhanden ist.

8. Zusammensetzung nach Anspruch 5, worin die Aktivsubstanz in einer medizinisch inerten Matrix in Form einer dreidimensionalen Struktur mit zumindest einem Oberflächenabschnitt enthalten ist und der Polymerkomplex auf dem zumindest einen Oberflächenabschnitt angeordnet ist.

9. Zusammensetzung nach Anspruch 8, worin die dreidimensionale Struktur ein Film ist.

10. Zusammensetzung nach Anspruch 5, worin das Gewichtsverhältnis von Polymerkomplex zu Aktivsubstanz 200.000:1 bis 1:100 beträgt.

11. Polymerkomplex-Zusammensetzung, umfassend (1) eine Kalziumpolycarbophil-Komponente, die ein in Wasser quellbares, jedoch wasserunlösliches, faseriges, vernetztes Polymer mit Carboxyl-Funktionalität ist, wobei das Polymer (a) eine Vielzahl an wiederkehrenden Einheiten, von denen zumindest 80% zumindest eine Carboxyl-Funktionalität enthalten, und (b) 0,05-1,5% Vernetzer frei von Polyalkenylpolyether enthält, wobei die Prozentangaben auf das Gewicht der wiederkehrenden, nicht polymerisierten Einheiten bzw. des Vernetzers bezogen sind, und (2) Wasser.

12. Zusammensetzung nach Anspruch 11, weiters umfassend einen oder mehrere Aktivsubstanzen.

13. Zusammensetzung nach Anspruch 11 oder 12, weiters umfassend ein oder mehrere Adjuvantien oder einen oder mehrere pharmazeutische Stabilisatoren.

14. Zusammensetzung nach Anspruch 11, 12 oder 13, worin die Zusammensetzung eine oder mehrere Komponenten enthält, die aus polaren und apolaren Co-Lösungsmitteln, Kohlenhydraten, Hydrokolloiden, Chelatbildnern, quaternären stickstoffhältigen Verbindungen, Konservierungsmitteln, Antioxidantien, Exzipienten, Zerfallsmodulatoren, Emulgatoren, Dispergatoren, Bindemitteln, Verdickern, künstlichen Süßstoffen, pilzhemmenden Mitteln, Farbstoffen, Geschmacksstoffen und Atemerfrischern ausgewählt sind.

15. Zusammensetzung nach Anspruch 12, worin die Aktivsubstanz aus Haushaltsmitteln, Kosmetika, Nährstoffen, Medikamenten oder landwirtschaftlichen Mitteln ausgewählt ist.

16. Zusammensetzung nach Anspruch 11, worin das Kalzium in einer Menge von 5-25%, vorzugsweise 18-22%, bezogen auf das Gewicht von Polycarbophil, vorhanden ist.

17. Zusammensetzung nach Anspruch 12, worin das Gewichtsverhältnis von Polymermatrix zu Aktivsubstanz von 200.000:1 bis 1:100 beträgt.

18. Polymersystem zur gesteuerten Freisetzung, umfassend den Polymerkomplex nach einem der Ansprüche 1 bis 17.

19. Verwendung der Zusammensetzung nach einem der Ansprüche 1 bis 17 zur Herstellung eines Medikaments zur Therapie unter gesteuerter Freisetzung.

20. Verwendung eines Polymersystems zur gesteuerten Freisetzung nach Anspruch 18 zur Herstellung eines Medikaments zur Therapie unter gesteuerter Freisetzung.

21. Verwendung von Kalziumpolycarbophil zur Herstellung eines geformten medizinischen Erzeugnisses zur Behandlung von Xerostomie.

22. Zusammensetzung, die sich zur Behandlung von Xerostomie eignet, umfassend ein Erzeugnis in einer Form, die sich zum Einführen in den Mund eines an Xerostomie leidenden Patienten eignet, wobei das Erzeugnis Kalziumpolycarbophil und 1 bis 10 ml Wasser umfaßt.

23. Verwendung der Zusammensetzung nach Anspruch 22 zur Herstellung eines Medikaments zur Behandlung von Xerostomie.

## Patentansprüche (Patentansprüche für folgende(n) Vertragsstaat(en): ES, GR)

1. Polymerkomplex-Zusammensetzung, umfassend einen Reaktionskomplex, gebildet durch die Wechselwirkung einer Kalziumpolycarbophil-Komponente (1), die ein in Wasser quellbares, jedoch wasserunlösliches, faseriges, vernetztes Polymer mit Carboxyl-Funktionalität ist, wobei das Polymer (a) eine Vielzahl an wiederkehrenden Einheiten, von denen zumindest 80% zumindest eine Carboxyl-Funktionalität enthalten, und (b) 0,05-1,5% Vernetzer frei von Polyalkenylpolyether enthält, wobei die Prozentangaben auf das Gewicht der wiederkehrenden, nicht polymerisierten Einheiten bzw. des Vernetzers bezogen sind, mit (2) Alginsäure oder einem Salz davon, wobei die Wechselwirkung in Gegenwart eines zweiwertigen Kations erfolgt.

2. Zusammensetzung nach Anspruch 1, worin das zweiwertige Kation Kalzium ist.

3. Zusammensetzung nach Anspruch 1 oder 2, worin die Polymerkomplex-Zusammensetzung weiters ein oder mehrere Adjuvantien oder einen oder mehrere pharmazeutische Stabilisatoren umfaßt.

4. Zusammensetzung nach einem der vorhergehenden Ansprüche, worin die Zusammensetzung eine oder mehrere weitere Komponenten enthält, die aus polaren und apolaren Co-Lösungsmitteln, Kohlenhydraten, Hydrokolloiden, Chelatbildnern, quaternären stickstoffhältigen Verbindungen, Konservierungsmitteln, Antioxidantien, Exzipienten, Zerfallsmodulatoren, Emulgatoren, Dispergatoren, Bindemitteln, Verdickern, künstlichen Süßstoffen, antifungalen Mitteln, Farbstoffen, Geschmacksstoffen und Atemerfrischern ausgewählt sind.

5. Zusammensetzung nach einem der vorhergehenden Ansprüche, worin die Polymerkomplex-Zusammensetzung weiters eine oder mehrere Aktivsubstanzen umfaßt.

6. Zusammensetzung nach Anspruch 5, worin die Aktivsubstanz aus Haushaltsmitteln, Kosmetika, Nährstoffen und landwirtschaftlichen Mitteln ausgewählt ist.

7. Zusammensetzung nach Anspruch 5, worin der Polymerkomplex in Form von zerkleinerten Teilchen vorliegt, die ein Sieb mit einer Größe von 100 Mesh (US Standard Sieve Series, Sieböffnung: 150 µm) passieren können, und die Aktivsubstanz als gesättigter gelöster Stoff in einem physiologisch annehmbaren, wäßrigen Träger vorhanden ist.

8. Zusammensetzung nach Anspruch 5, worin die Aktivsubstanz in einer medizinisch inerten Matrix in Form einer dreidimensionalen Struktur mit zumindest einem Oberflächenabschnitt enthalten ist und der Polymerkomplex auf dem zumindest einen Oberflächenabschnitt angeordnet ist.

9. Zusammensetzung nach Anspruch 8, worin die dreidimensionale Struktur ein Film ist.

10. Zusammensetzung nach Anspruch 5, worin das Gewichtsverhältnis von Polymerkomplex zu Aktivsubstanz 200.000:1 bis 1:100 beträgt.

11. Polymerkomplex-Zusammensetzung, umfassend (1) eine Kalziumpolycarbophil-Komponente, die ein in Wasser quellbares, jedoch wasserunlösliches, faseriges, vernetztes Polymer mit Carboxyl-Funktionalität ist, wobei das Polymer (a) eine Vielzahl an wiederkehrenden Einheiten, von denen zumindest 80% zumindest eine Carboxyl-Funktionalität enthalten, und (b) 0,05-1,5% Vernetzer frei von Polyalkenylpolyether enthält, wobei die Prozentangaben auf das Gewicht der wiederkehrenden, nicht polymerisierten Einheiten bzw. des Vernetzers bezogen sind, und (2) Wasser.

12. Zusammensetzung nach Anspruch 11, weiters umfassend einen oder mehrere Aktivsubstanzen.

13. Zusammensetzung nach Anspruch 11 oder 12, weiters umfassend ein oder mehrere Adjuvantien oder einen oder mehrere pharmazeutische Stabilisatoren.

14. Zusammensetzung nach Anspruch 11, 12 oder 13, worin die Zusammensetzung eine oder mehrere weitere Komponenten enthält, die aus polaren und apolaren Co-Lösungsmitteln, Kohlenhydraten, Hydrokolloiden, Chelatbildnern, quaternären stickstoffhältigen Verbindungen, Konservierungsmitteln, Antioxidantien, Exzipienten, Zerfallsmodulatoren, Emulgatoren, Dispergatoren, Bindemitteln, Verdickern, künstlichen Süßstoffen, antifungalen Mitteln, Farbstoffen, Geschmacksstoffen und Atemerfrischern ausgewählt sind.

15. Zusammensetzung nach Anspruch 12, worin die Aktivsubstanz aus Haushaltsmitteln, Kosmetika, Nährstoffen und landwirtschaftlichen Mitteln ausgewählt ist.

16. Zusammensetzung nach Anspruch 11, worin das Kalzium in einer Menge von 5-25%, vorzugsweise 18-22%, bezogen auf das Gewicht von Polycarbophil, vorhanden ist.

17. Zusammensetzung nach Anspruch 12, worin das Gewichtsverhältnis von Polymermatrix zu Aktivsubstanz von 200.000:1 bis 1:100 beträgt.

18. Polymersystem zur gesteuerten Freisetzung, umfassend den Polymerkomplex nach einem der Ansprüche 1 bis 17.

19. Verwendung der Zusammensetzung nach einem der Ansprüche 1 bis 17 zur Herstellung eines Medikaments zur Therapie unter gesteuerter Freisetzung.

20. Verwendung eines Polymersystems zur gesteuerten Freisetzung nach Anspruch 18 zur Herstellung eines Medikaments zur Therapie unter gesteuerter Freisetzung.

21. Verwendung von Kalziumpolycarbophil zur Herstellung eines geformten medizinischen Erzeugnisses zur Behandlung von Xerostomie.

22. Verwendung einer Zusammensetzung, umfassend ein Erzeugnis in einer Form, die sich zum Einführen in den Mund eignet, wobei das Erzeugnis Kalziumpolycarbophil und 1 bis 10 ml Wasser umfaßt, um ein Medikament zur Behandlung von Xerostomie herzustellen.

23. Verfahren zur Herstellung einer pharmazeutischen Zusammensetzung, umfassend das Formulieren einer Zusammensetzung, die ein Medikament und eine Polymerkomplex-Zusammensetzung nach einem der Ansprüche 1-4, 11, 14 oder 16 umfaßt.

24. Verfahren nach Anspruch 23, worin der Polymerkomplex in Form von zerkleinerten Teilchen vorliegt, die ein Sieb mit einer Größe von 100 Mesh (US Standard Sieve Series, Sieböffnung: 150 µm) passieren können, und das Medikament als gesättigter gelöster Stoff in einem physiologisch annehmbaren, wäßrigen Träger vorhanden ist.

25. Verfahren nach Anspruch 23, das eine Zusammensetzung ergibt, worin das Medikament in einer medizinisch inerten Matrix in Form einer dreidimensionalen Struktur mit zumindest einem Oberflächenabschnitt enthalten ist und der Polymerkomplex auf dem zumindest einen Oberflächenabschnitt angeordnet ist.

26. Verfahren nach Anspruch 25, worin die dreidimensionale Struktur ein Film ist.

27. Verfahren nach Anspruch 25, worin das Gewichtsverhältnis von Polymerkomplex zum Medikament 200.000:1 bis 1:100 beträgt.

28. Verfahren zur Herstellung einer pharmazeutischen Zusammensetzung aus einer Polymerkomplex-Zusammensetzung, umfassend (1) eine Kalziumpolycarbophil-Komponente, die ein in Wasser quellbares, jedoch wasserunlösliches, faseriges, vernetztes Polymer mit Carboxyl-Funktionalität ist, wobei das Polymer (a) eine Vielzahl an wiederkehrenden Einheiten, von denen zumindest 80% zumindest eine Carboxyl-Funktionalität enthalten, und (b) 0,05-1,5% Vernetzer frei von Polyalkenylpolyether enthält, wobei die Prozentangaben auf das Gewicht der wiederkehrenden, nicht polymerisierten Einheiten bzw. des Vernetzers bezogen sind, und (2) Wasser, sowie aus einem Medikament.

29. Verfahren nach Anspruch 28, worin die pharmazeutische Zusammensetzung ein oder mehrere Adjuvantien oder einen oder mehrere pharmazeutische Stabilisatoren enthält.

30. Verfahren nach Anspruch 28 oder 29, worin die pharmazeutische Zusammensetzung eine oder mehrere weitere Komponenten enthält, die aus polaren und apolaren Co-Lösungsmitteln, Kohlenhydraten, Hydrokolloiden, Chelatbildnern, quaternären stickstoffhältigen Verbindungen, Konservierungsmitteln, Antioxidantien, Exzipienten, Zerfallsmodulatoren, Emulgatoren, Dispergatoren, Bindemitteln, Verdickern, künstlichen Süßstoffen, antifungalen Miileln, Farbstoffen, Geschmacksstoffen und Atemerfrischern ausgewählt sind.

31. Verfahren nach Anspruch 28, worin das Kalzium in einer Menge von 5-25%, vorzugsweise 18-22%, bezogen auf das Gewicht von Polycarbophil, vorhanden ist.

32. Verfahren nach Anspruch 28, worin das Gewichtsverhältnis von Polymermatrix zum Medikament von 200.000:1 bis 1:100 beträgt.

## Revendications (Revendications pour l'(les) Etat(s) contractant(s) suivant(s): AT, BE, CH, LI, DE, DK, FR, GB, IT, LU, NL, SE)

1. Composition de complexe polymérique comprenant un complexe réactionnel formé par l'interaction de (1) un composant calcium polycarbophile qui est un polymère gonflable dans l'eau, mais insoluble dans l'eau, fibreux, à fonctionnalité carboxy réticulé , ledit polymère contenant (a) une pluralité d'unités récurrentes dont au moins 80 pour cent contiennent au moins une fonctionnalité carboxyle, et (b) 0,05 à 1,5 pour cent d'agent de réticulation exempt de polyalkényl polyétner, lesdits pourcentages étant basés sur les poids de l'unité récurrente non polymérisée et de l'agent de réticulation, respectivement,avec (2) de l'acide alginique ou un de ses sels, ladite interaction étant effectuée en présence d'un cation divalent.

2. Composition selon la revendication 1, où le cation divalent est le calcium.

3. Composition selon la revendication 1 ou 2, où ladite composition de complexe polymérique comprend, de plus, un ou plus adjuvants ou un ou plus stabilisants pharmaceutiques.

4. Composition selon l'une quelconque des revendications précédentes, où la composition contient un ou plus composants supplémentaires choisis par mi las cosolvants polaires et non polaires, les carbohydrates, les hydrocolloïdes, les agents chélatants, les composés contenant un azote quaternaire, les conservateurs, les antioxydants, les excipients, les agents modulant la désintégration, les émulsifiants, les dispersants, les liants, les épaississants, les édulcorants artificiels, les agents antifongiques, les agents colorants, les arômes, et les rafraîchisseurs d'haleine.

5. Composition selon l'une quelconque des revendications précédentes, où ladite composition de complexe polymérique comprend de plus un ou plusieurs agents actifs.

6. Composition selon la revendication 5, où l'agent actif est choisi parmi un agent ménager, un agent cosmétique, un agent nutritionnel, un médicament et un agent d'agriculture.

7. Composition selon la revendication 5, où ledit complexe polymérique est sous la forme de particules broyées en petits morceaux capables de passer à travers les mailles d'un tamis ayant une taille de 100 mesh, de la Série des Tamis Standard US (ouverture du tamis : 150 µm) et ledit agent actif est présent sous la forme d'un soluté saturé dans un porteur aqueux physiologiquement acceptable.

8. Composition selon la revendicaiton 5, où ledit agent actif est contenu dans une matrice inerte médicalement sous la forme d'une structure tridimensionnelle ayant au moins une portion de surface, et ledit complexe polymérique est disposé sur au moins ladite une portion de surface.

9. Composition selon la revendication 8, où ladite structure tridimensionnelle est un film.

10. Composition selon la revendication 5, où le rapport en poids dudit complexe polymérique audit agent actif est de 200 000:1 à 1:100.

11. Composition de complexe polymérique comprenant (1) un composant calcium polycarbophile qui est un polymère gonflable dans l'eau, mais insoluble dans l'eau, fibreux, à fonctionnalité carboxy réticulé, ledit polymère contenant (a) une pluralité d'unités récurrentes dont au moins 80 pour cent contiennent au moins une fonctionnalité carboxy, et (b) 0,05 à 1,5 pour cent d'agent de réticulation exempt de polyalkényl polyéther, lesdits pourcentages étant sur la base des poids de l'unité récurrente non polymérisée et de l'agent de réticulation, respectivement, avec (2) de l'eau.

12. Composition selon la revendication 11, comprenant de plus un ou plus agents actifs.

13. Composition selon la revendication 11 ou 12, comprenant de plus un ou plus adjuvants ou un ou plus stabilisants pharmaceutiques.

14. Composition selon la revendication 11, 12 ou 13, où la composition contient un ou plus composants supplémentaires choisis parmi les cosolvants polaires et non polaires, les carbohydrates, les hydrocolloïdes, les agents chélatants, les composés contenant un azote quaternaire, les conservateurs, les antioxydants, les excipients, les agents modulant la désintégration, les émulsifiants, les dispersants, les liants, les épaississants, les édulcorants artificiels, les agents antifongiques, les agents colorants, les arômes, et les rafraîchisseurs d'haleine.

15. Composition selon la revendication 12, où l'agent actif est choisi parmi un agent ménager, un agent cosmétique, un agent nutritionnel, un médicament ou un agent pour l'agriculture.

16. Composition selon la revendication 11, où le calcium est présent en une quantité de 5 à 25 pour cent et, de préférence, de 18 à 22 pour cent, sur la base du poids du polycarbophile.

17. Composition selon la revendication 12, où le rapport en poids de ladite matrice polymérique audit agent actif est de 200 000:1 à 1:100.

18. Système polymérique à libération contrôlée comprenant le complexe polymérique de l'une des revendications 1 à 17.

19. Utilisation de la composition selon l'une des revendications 1 à 17, pour la préparation d'un médicament pour une thérapie à libération contrôlée.

20. Utilisation du système à libération contrôlée polymérique selon la revendication 18, pour la préparation d'un médicament pour la thérapie à libération contrôlée.

21. Utilisation de calcium polycarbophile dans la fabrication d'un article médical mis en forme pour traiter la xérostomie.

22. Composition utile dans le traitement de la xérostomie comprenant un article d'une forme appropriée pour insertion dans la bouche d'un patient souffrant de xérostomie, ledit article comprenant du calcium polycarbophile et de 1 à 10 ml d'eau.

23. Utilisation de la composition selon la revendication 22, pour la préparation d'un médicament pour traiter la xérostomie.

## Revendications (Revendications pour l'(les) Etat(s) contractant(s) suivant(s): ES, GR)

1. Composition de complexe polymérique comprenant un complexe réactionnel formé par l'interaction de (1) un composant calcium polycarbophile qui est un polymère à fonction carboxy réticulé, fibreux, gonflable dans l'eau, mais insoluble dans l'eau, ledit polymère contenant (a) une pluralité d'unités récurrentes dont au moins 80 pourcent contiennent au moins une fonctionnalité carboxyl, et (b) de 0,05 à 1,5 pourcent d'agent de réticulation exempt de polyalkényl polyéther, lesdits pourcentages étant basés sur les poids de l'unité récurrente non polymérisée et de l'agent de réticulation, respectivement avec (2) l'acide alginique ou un sel de celui-ci, ladite interaction étant effectuée en présence d'un cation divalent.

2. Composition selon la revendication 1 où le cation divalent est le calcium.

3. Composition selon la revendication 1 ou 2 dans laquelle ladite composition de complexe polymérique comprend de plus un ou plus adjuvants ou un ou plus stabilisants pharmaceutiques.

4. Composition selon l'une quelconque des revendications précédentes dans laquelle la composition contient de plus des composants supplémentaires choisis parmi les cosolvants polaires et non polaires, les carbohydrates, les hydrocolloïdes, les agents de chélation, les composés contenant un azote quaternaire, les conservateurs, les antioxydants, les excipients, les agents de modulation de la désintégration, les émulsifiants, les dispersants, les liants, les épaississants, les édulcorants artificiels, les agents antifongiques, les agents colorants, les arômes et les rafraîchisseurs d'haleine.

5. Composition selon l'une quelconque des revendications précédentes où ladite composition de complexe polymérique comprend de plus un ou plus agents actifs.

6. Composition selon la revendication 5, où l'agent actif est choisi parmi un agent ménager, un agent cosmétique, un agent nutritionnel, et un agent d'agriculture.

7. Composition selon la revendication 5, où ledit complexe polymérique est sous la forme de particules réduites en morceaux capables de passer à travers l'écran d'un tamis ayant une taille de 100 mesh, de la série des tamis Standard U.S. (ouverture de tamis : 150 µm) et ledit agent actif est présent en tant que soluté saturé dans un porteur aqueux physiologiquement acceptable.

8. Composition selon la revendication 5, où ledit agent actif est contenu dans une matrice médicalement inerte sous la forme d'une structure tridimensionnelle ayant au moins une portion de surface, et ledit complexe polymérique est disposé sur au moins ladite une portion de surface.

9. Composition selon la revendication 8, où ladite structure tridimensionnelle est un film.

10. Composition selon la revendication 5 où le rapport en poids dudit complexe polymérique audit agent actif est de 200 000:1 à 1:100.

11. Composition de complexe polymérique comprenant (1) un composant calcium polycarbophile qui est un polymère à fonction carboxy réticulé, fibreux, gonflable dans l'eau, mais insoluble dans l'eau, ledit polymère contenant (a) une pluralité d'unités récurrentes dont au mains 80 pourcent contiennent au moins une fonctionnalité carboxyle, et (b) de 0,05 à 1,5 pourcent d'agent de réticulation exempt de polyalkényl polyéther, lesdits pourcentages étant basés sur les poids de l'unité récurrente non polymérisée et de l'agent de réticulation, respectivement avec (2) de l'eau.

12. Composition selon la revendication 11 comprenant de plus un ou plus agents actifs.

13. Composition selon la revendication 11 ou 12 comprenant de plus un ou plus adjuvants ou un ou plus stabilisants pharmoceutiques.

14. Composition selon la revendication 11, 12 ou 13 où la composition contient un ou plus composants supplémentaires choisis parmi les cosolvants polaires et non polaires, les carbohydrates, les hydrocolloïdes, les agents de chélation, les composés contenant un azote quaternaire, les conservateurs, les antioxydants, les excipients, les agents de modulation de la désintégration, les émulsifiants, les dispersants, les liants, les épaississants, les éducorants artificiels, les agents antifongiques, les agents colorants, les arômes et les rafraîchisseurs d'haleine.

15. Composition selon la revendication 12 où l'agent actif est choisi parmi un agent ménager, un agent cosmétique, un agent nutritionnel et un agent d'agriculture.

16. Composition selon la revendication 11 où le calcium est présent une quantité de 5 à 25 % et de préférence de 18 à 22 pourcent, sur la base du poids du polycarbophile.

17. Composition selon la revendication 12 où le rapport en poids de ladite matrice polymérique audit agent actif est de 200 000:1 à 1:100.

18. Système à libération contrôlée polymérique comprenant le complexe polymérique de l'une quelconque des revendications 1 à 17.

19. Utilisation de la composition selon l'une quelconque des revendications 1 à 17 pour la préparation d'un médicament pour une thérapie à libération contrôlée.

20. Utilisation d'un système de libération contrôlée polymérique selon la revendication 18 pour la préparation d'un médicament pour une thérapie à libération contrôlée.

21. Utilisation de calcium polycarbophile dans la fabrication d'un article médical mis en forme pour traiter la xérostomie.

22. Utilisation d'une composition comprenant un article en une forme appropriée pour insertion dans la bouche, ledit article comprenant du calcium polycarbophile et de 1 à 10 ml d'eau, pour la préparation d'un médicament pour traiter la xérostomie.

23. Procédé pour préparer une composition pharmaceutique comprenant la formulation d'une composition qui comprend un médicament et une composition de complexe polymérique selon l'une quelconque des revendications 1 à 4, 11, 14 ou 16.

24. Procédé selon la revendication 23 dans lequel ledit complexe polymérique est sous la forme de particules réduites en morceaux capables de passer à travers l'écran d'un tamis d'un tamis ayant une taille de 100 mesh, de la série des tamis Standard U.S., (ouverture du tamis : 150 µm) et où ledit médicament est présent en tant que soluté saturé dans un porteur aqueux physiologiquement acceptable.

25. Procédé selon le revendication 23 qui donne une composition dans laquelle ledit médicament est contenu dans une matrice médicalement inerte sous la forme d'une structure tridimensionnelle ayant au moins une portion de surface, et ledit complexe polymérique est disposé sur au moins ladite une portion de surface.

26. Procédé selon la revendication 25 où ladite structure tridimensionnelle est un film.

27. Procédé selon la revendication 5 où le rapport en poids dudit complexe polymérique audit médicament est de 200 000:1 à 1:100.

28. Procédé pour préparer une composition pharmaceutique d'une composition de complexe polymérique comprenant (1) un composent calcium polycarbophile et un polymère à fonction carboxy réticulé, fibreux, gonflable dans l'eau, mais insoluble dans l'eau, ledit polymère contenant (a) une pluralité d'unités récurrentes dont au moins 80 pourcent contiennent au moins une fonctionnalité carboxyle, et (b) de 0,05 à 1,5 pourcent d'agent de réticulation exempt de polyalkenyl polyéther, lesdits pourcentages étant basés sur les poids de l'unité récurrente non polymérisée et de l'agent de réticulation, respectivement avec (2) de l'eau ; et un médicament.

29. Procédé selon la revendication 28 dans lequel la composition pharmaceutique contient un ou plus adjuvants ou un ou plus stabilisants pharmaceutiques.

30. Procédé selon la revendication 28 ou 29 dans lequel la composition pharmaceutique contient un ou plus composants supplémentaires choisis parmi les cosolvants polaires et non polaires, les carbohydrates, les hydrocolloïdes, les agents de chélation, les composés contenant un azote quaternaire, les conservateurs, les antioxydants, les excipients, les agents modulant la désintégration, les émulsifiants, les dispersants, les liants, les épaississants, les édulcorants artificiels, les agents antifongiques, les agents colorants, les arômes, et les rafraîchisseurs d'haleine.

31. Procédé selon la revendication 28 dans lequel la calcium est présent en une quantité de 5 à 25 pourcent et de préférence de 18 à 22 pourcent sur la base du poids du polycarbophile.

32. Procédé selon la revendication 28 dans lequel le rapport en poids de ladite matrice polymérique audit médicament est de 200 000:1 à 1:100.
